# EUROPEAN PATENT SPECIFICATION

(11) **EP 2 205 742 B1**
(45) Date of publication and mention of the grant of the patent: **09.11.2011**
(21) Application number: 08837232.1
(22) Date of filing: 08.10.2008
(51) Int. Cl.: C12N 15/11, C12N 5/0781

(54) **B CELL TOLERANCE INDUCTION**
B-ZELLEN-TOLERANZINDUKTION
INDUCTION D'UNE TOLÉRANCE AUX LYMPHOCYTES B

(30) Priority: 08.10.2007 US 978343 P
(43) Date of publication of application: 14.07.2010
(73) Proprietor: VIB VZW, 9052 Gent (BE); Life Sciences Research Partners VZW, 3000 Leuven (BE)
(72) Inventor: VANDENDRIESSCHE, Thierry, B-3360 Lovenjoel (BE); CHUAH, Marinee, B-3360 Lovenjoel (BE); SAINT-REMY, Jean-Marie, B-1390 Grez-doiceau (BE); JACQUEMIN, Marc, B-5330 Sart-bernard (BE)
(86) International application number: PCT/EP2008/063477
(87) International publication number: WO 2009/047270

(56) References cited:
- MIZOGUCHI ATSUSHI ET AL: "A case for regulatory B cells" JOURNAL OF IMMUNOLOGY, vol. 176, no. 2, January 2006 (2006-01), pages 705-710, XP002510150 ISSN: 0022-1767
- BURKE FIONA ET AL: "IL-10-producing B220+ CD11c- APC in mouse spleen" JOURNAL OF IMMUNOLOGY, vol. 173, no. 4, 15 August 2004 (2004-08-15), pages 2362-2372, XP002510151 ISSN: 0022-1767 cited in the application
- MIZOGUCHI ATSUSHI ET AL: "Chronic intestinal inflammatory condition generates IL-10-producing regulatory B cell subset characterized by CD1d upregulation" IMMUNITY, vol. 16, no. 2, February 2002 (2002-02), pages 219-230, XP002510152 ISSN: 1074-7613
- LEI ET AL: "Tolerance induction via a B-cell delivered gene therapy-based protocol: Optimization and role of the Ig scaffold" CELLULAR IMMUNOLOGY, ACADEMIC PRESS, SAN DIEGO, CA, US, vol. 235, no. 1, 1 May 2005 (2005-05-01), pages 12-20, XP005109963 ISSN: 0008-8749 cited in the application
- LAMPROPOULOU VICKY ET AL: "TLR-activated B cells suppress T cell-mediated autoimmunity." JOURNAL OF IMMUNOLOGY (BALTIMORE, MD. : 1950) 1 APR 2008, vol. 180, no. 7, 1 April 2008 (2008-04-01), pages 4763-4773, XP002510153 ISSN: 0022-1767
- BARR TOM A ET AL: "TLR-mediated stimulation of APC: Distinct cytokine responses of B cells and dendritic cells" EUROPEAN JOURNAL OF IMMUNOLOGY, vol. 37, no. 11, November 2007 (2007-11), pages 3040-3053, XP002510154 ISSN: 0014-2980
- DIMARTINO ET AL: "Insoluble immune complexes are most effective at triggering IL-10 production in human monocytes and synergize with TLR ligands and C5a" CLINICAL IMMUNOLOGY, ACADEMIC PRESS, US, vol. 127, no. 1, 16 January 2008 (2008-01-16), pages 56-65, XP022535313 ISSN: 1521-6616

## Description

The present invention relates to the induction of immune tolerance, by a novel method of inducing IL-10 production in B cells, whereby said B cells can be loaded with antigen before, during or after the induction of the IL-10 production. More specifically, the invention relates to the transduction of the B cell with a retroviral vector encoding an antigen, whereby the retroviral particle, in combination with its cognate viral receptor activates the TLR2 receptor and hereby induces IL-10 production in the B cell.

Establishing long-term antigen-specific unresponsiveness in a fully immunocompetent host is required to effectively treat autoimmune diseases and allergy and to prevent or suppress cellular and/or humoral immune responses following allogeneic transplantation and gene therapy. Maintenance of antigen-specificity typically requires presentation of the antigen within the context of major histocompatibility complex class II determinants (MHC-II) on antigen-presenting cells (APC). However, exposure of APC to exogenous antigens often results in the concomitant expression of co-stimulatory signals and ultimately in T cell activation, which, consequently, precludes induction of immune tolerance. Conversely, the lack of co-stimulatory signals on APCs may confer antigen-specific unresponsiveness (Cassell and Schwartz, 1994). This could possibly be achieved by using genetically modified APC that present the antigenic peptide in association with MHC-II, while preventing expression of such co-stimulatory molecules.

Dendritic cells are known to induce antigen-specific unresponsiveness or boost an immune response, depending on their state of maturation (Chaussabel and Banchereau, 2005; Mahnke and Enk, 2005), which makes them particularly attractive for genetic modification. However, genetic modification of dendritic cells often results in the upregulation of costimulatory molecules and MHC-II with a concomitant increase in their immune stimulatory properties (Korst et al., 2002). Moreover, their life-span is limited in vivo since myeloid cells turnover relatively rapidly compared to lymphoid cells (Lee et al., 1998).

B lymphocytes offer an attractive alternative, due to their ability to survive for long periods of time after adoptive transfer into a recipient host. Moreover, they efficiently interact with T cells within lymph nodes and splenic germinal centers. However, the use of genetically modified B cells for immuno-modulation has initially been hampered by relatively poor gene transfer efficiencies and by their ability to tolerate only naive but not primed T cell (Fuchs and Matzinger, 1992).

The low gene transfer efficiencies in primary B cells could be overcome using Moloney murine leukemia virus (MLV)-based retroviral vectors instead. Indeed, we and others have shown that relatively high stable transduction efficiencies (>50%) could be achieved, following retroviral transduction of primary murine B cells (Janssens et al., 2003b; Lei and Scott, 2005). In addition, Scott and coworkers recently showed that genetically-modified B cells could be used to tolerize not only naïve but also primed T cells. Indeed, B cells transduced with a retroviral vector expressing domains of coagulation factor VIII (FVIII) linked to an IgG heavy chain scaffold, suppress the ongoing production of anti-FVIII antibodies upon transfer to a FVIII-immunized host (Lei and Scott, 2005). The molecular and cellular mechanisms that contributed to this immune unresponsiveness are not fully understood, but the authors claim that the tolerance induced by the IgG peptide fusion is IL-10 independent (EI-Amine et al, 2000). Moreover, the IgG scaffold is considered as obligatory to induce this IL-10 independent immune tolerance (Lei et al, 2005).

To address some of these outstanding questions and unravel the mechanisms that contribute to the induction of immune tolerance using B cells, we evaluated whether retrovirally transduced B cells, could confer antigen-specific unresponsiveness using a novel approach, after transfer to a naive or primed host. In particular, we wanted to test the hypothesis whether direct intracellular targeting of an antigen to late endosomes for MHC-II presentation, would endow B cells with tolerogenic properties on CD4+ specific T cells, without having to rely on a lg heavy chain scaffold.

As a model antigen, encoded by the retroviral vector, we used a peptide encompassing amino acid residues 21 to 35 (p21-35) from a major group 2 allergen from the house dust mite *Dermatophagoides pteronyssinus,* Der p 2, and involved in the development of allergic diseases such as asthma. p21-35 has been extensively studied in our laboratory: it contains both a major promiscuous T cell epitope and a dominant B epitope (Wu et al., 2002), thereby providing a suitable sequence for the study of tolerance induction at B and T cell levels. Injection of p21-35 in conventional adjuvant elicits strong specific antibody responses, while the same peptide under tolerogenic conditions results in prevention and partial suppression of the immune response towards Der p 2 (Wu et al., 2000).

To ensure efficient presentation of the antigen in the context of MHC-II to CD4+ T helper cells, the p21-35 transgene was linked to the carboxyl-terminal end (amino acid 488-539) of the melanosomal glycoprotein 75 (gp75). Gp75 contains a dileucine-based sorting and retention motif, which serves as a melanosomal transport signal (MTS) that is crucial for targeting proteins to the late endosomes and the MHC-II pathway (Liu et al., 2001). Surprisingly, we found that a long-lasting antigen-specific immune tolerance can be established using genetically modified B cells designed to express a potent allergenic peptide in association with MHC-II, without a treatment of the B cells with LPS or any other additional activation step. The genetically modified B-cells effectively prevented and suppressed the antigen-specific humoral immune response. Even more surprisingly, the tolerance induction is dependent on cell surface recruitment of Toll-like receptor 2 (TLR2) and the retroviral cellular receptor (mCAT1) into lipid microrafts, leading ultimately to constitutive IL-10 production by transduced B cells and induction of type 1-like regulatory T cells.

Constitutive IL-10 induction was mediated by an epigenetic control mechanism that required activation of the STAT-3 transcription factor and increased binding of phosphorylated histone 3 on the corresponding STAT-3 binding site in the IL-10 promoter. For the first time it is demonstrated that endosomal retargeting in B-cells can induce long-lasting immune tolerance in both prophylactic and therapeutic settings.

A first aspect of the invention is a method to condition B cells to produce IL-10, comprising activation of the TLR2 receptor, in combination with the activation of a second transmembrane protein. Activation as used here means the binding of a ligand to the receptor or transmembrane protein, resulting in an intracellular signal, preferably an activation of the STAT-3 transcription factor. A ligand as used here can be any compound, including but not limited to chemical molecules, proteins, glycoproteins, or protein complexes. Preferably the second membrane protein is a transporter protein. Even more preferably, said second membrane protein is a transporter protein that is used as retroviral receptor. Such proteins are known to the person skilled in the art and include, but are not limited to PiT1, PiT2, ASCT1, ASCT2, SLC35F2 and mCAT1. Most preferably, said second membrane protein is mCAT1. The activation of the TLR2 receptor, in combination with the activation the transmembrane protein can be achieved by simultaneous binding of two different ligands to the two proteins, or it can be by the binding of one ligand to the two proteins. Preferably, one ligand activates both receptors. In one preferred embodiment, said ligand is a retroviral envelope, preferably an MLV envelope or any envelope used for pseudotyping MLV (including but not limited to 10A1, GALV, VSV-G, RD114, HBV, HCV, MLV-A, MLV-E, Ebo-Z, LCMV, RRV). In another preferred embodiment, said ligand is a bispecific antibody, binding and activating both receptors. An antibody can be any type of antibody known the person skilled in the art, including but not limited to conventional bispecific antibodies, single chain antibodies and camelid derived antibodies such as nanobodies.

B cells, conditioned to produce IL-10 according to the invention can be loaded with antigen, to obtain an antigen presenting, immune tolerance-inducing cell.. Loading with the antigen can be realized before, during or after the conditioning. Indeed, an antigen presenting B-cell can be conditioned to produce IL-10 by exposing it to, as a non limiting example, a MLV particle or envelope or with a bispecific TLR2 activating / mCAT1 activating antibody. Alternatively, the antigen to be presented and the activating ligand or ligands are presented to the B cell at the same time. In another embodiment, the IL-10 activation occurs first, followed by the presentation of the antigen. A special case of the latter embodiment is the transduction with a retroviral vector, whereby the viral particle interacts with the TLR2 and its retroviral receptor, resulting in IL-10 induction, followed by expressing and presentation of the antigen on the B-cell. Another aspect of the invention is a method to condition B cells to produce IL-10, comprising colocalization of the TLR2 receptor in combination with a second transmembrane protein or with another membrane component into lipid rafts. Preferably, said other membrane component is the VSV-G cellular receptor. Preferably, said colocalization is realized by a protein or a particle that binds and activates the TLR2 receptor and binds to the second membrane protein or the other membrane component.

Herein described is also a method to produce IL-10 producing B cells, inducing Ig-scaffold independent immune tolerance against an antigen, preferably IgG-scaffold independent immune tolerance comprising retroviral transduction of the B cell with a gene encoding said antigen. Ig scaffold, as used here, is the sequence comprising, preferably consisting of the V_{H} of an antibody, whereby said sequence can be fused to an antigen. The fusion of the antigen to the scaffold can be amino or carboxy terminal, or the antigen is fused into the scaffold, such as, as a non-limiting example, after the first framework region.

IgG-scaffold independent tolerance, as used here, means that the antigen used for presentation on the B cells is not fused to an IgG-scaffold. Indeed, IgG fused antigens, expressed in B cells may induce tolerance, but in an IL-10 independent way (EI-Amine et al, 2000); the lg-scaffold independent and/or IgG-scaffold independent immune tolerance is IL-10 dependent. In view of these results, an IgG independent, IL-10 dependent tolerance induction by B cells is unexpected. The retroviral transduction can be ex vivo or in vivo. Indeed, B cells can be isolated, transduced and reintroduced in the host. Alternatively, the B cells are targeted by the retroviral vector and transduced in vivo. Methods for targeting and transducing B cells are known to the person skilled in the art and have been described, as a non limiting example, by Chandrashekran et al, 2004 and by Yang et al., 2006.

Also described is a method to induce Ig independent immune tolerance, preferably IgG independent immune tolerance against an antigen in a mammal, comprising (i) isolating B cells from said mammal (ii) transducing said B cells with a retroviral vector, encoding said antigen and (iii) reintroducing the transduced B cells in said mammal. Preferably, said retroviral vector is packed in a viral envelope that interacts with both TLR2 and mCAT1.

B-cells, conditioned according to the invention, are used to treat immune and/or autoimmune diseases. Auto-immune diseases as used here include, but are not limited to Multiple sclerosis, rheumatoid arthritis, allergic asthma and diabetes.

### BRIEF DESCRIPTION OF THE FIGURES

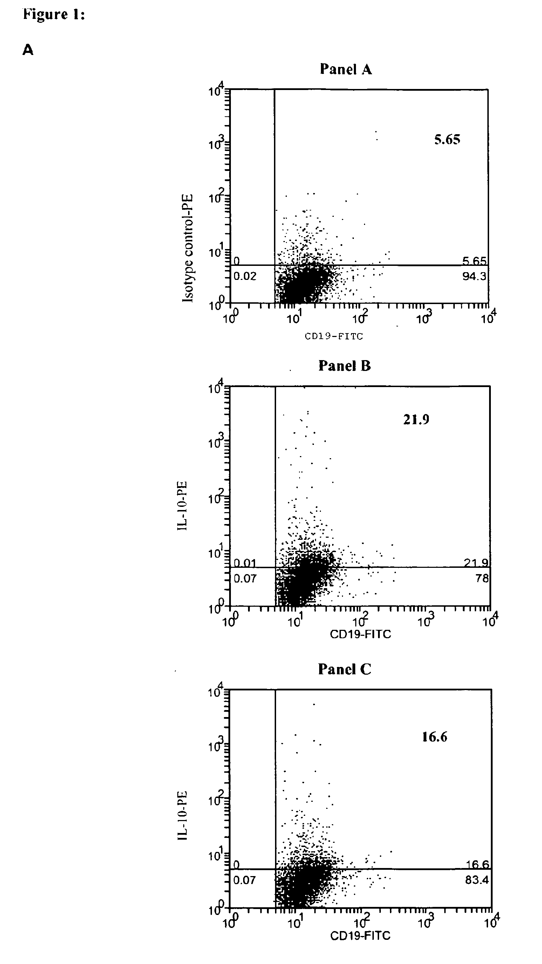

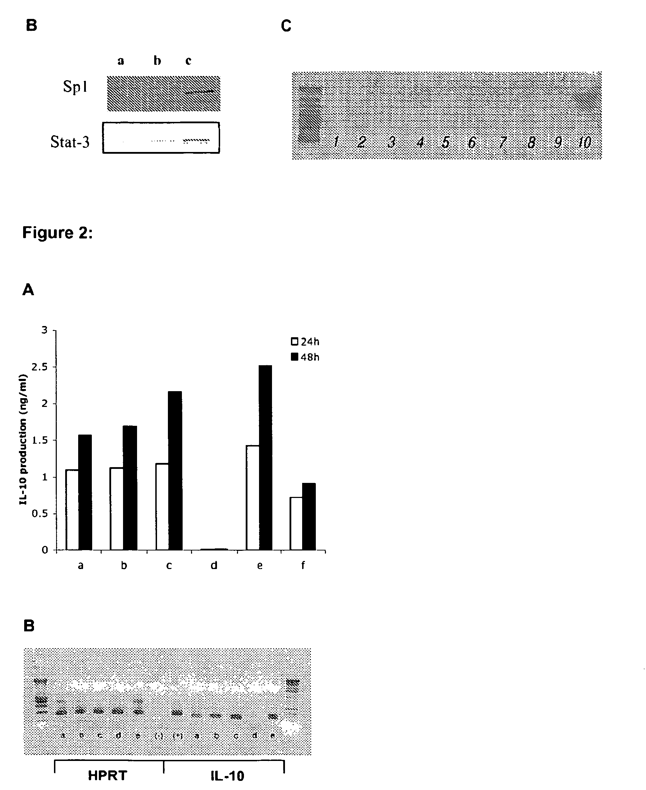

### Figure 1. Production of IL-10 by p21 gp75 transduced B cells

A. The intracellular production of IL-10 in CD19⁺ cells was assessed by FACS analysis in B cells activated by LPS and transduced (panel B) and in B cells activated by LPS only (panel C). Percentages of cells positive for IL-10 expression are shown together with the isotype control on LPS activated B cells (panel A); B. Western blot photograph showing phosphorylation of SP1 and STAT-3 in p21gp75 transduced B cells, nuclear protein was extracted from resting (a), LPS-activated (b) and p21gp75 transduced B cells (c), SP1 and STAT-3 proteins were immunoblotted with anti-SP1 and anti STAT-3 antibodies. C. CHIP analysis of histone 3 (H3) modifications at the IL-10 promoter site for STAT3 binding in resting, LPS-activated and transduced B cells. Immunoprecipitations were performed using Abs specific to acetylated H3 (line 5-7) or phosphorylated H3 (line 8-10), and conventional semi-quantitative PCR was performed using primers specific for STAT3. From left: MW marker (100 bp DNA ladder); lane 1, negative control, lanes 2-4: input DNA from resting, LPS-activated and transduced B cells respectively; lanes 5-7: STAT3 specific PCR product for acetylated H3; and lanes 8-10 STAT3 transcript for phosphorylated H3.

### Figure 2. IL-10 transcription is mediated by TLR2 signaling

B cells activated by LPS and incubated in the presence of antibodies to either CD180 (a), TLR3 (c), TLR2 (d) prior and during transduction with the MND-p21gp75 vector. Controls included an IgG2a isotype control antibody (b), no antibody (e) and LPS-activated B-cells and not transduced (f) **A.** Concentrations of IL-10 as evaluated by ELISA in cell supernatants. Results obtained after 24h and 48 h are expressed in ng/ml **B**. RT-PCR analysis showing the presence of IL-10 transcripts. Lane 1: MW marker (smart ladder); lanes 2-6: HPRT for groups a to e, respectively; (-) and (+): negative and positive control for IL-10, respectively; lanes 9-13: IL-10 transcript for groups a to e, respectively. Iane 14: MW marker (100 bp DNA ladder).

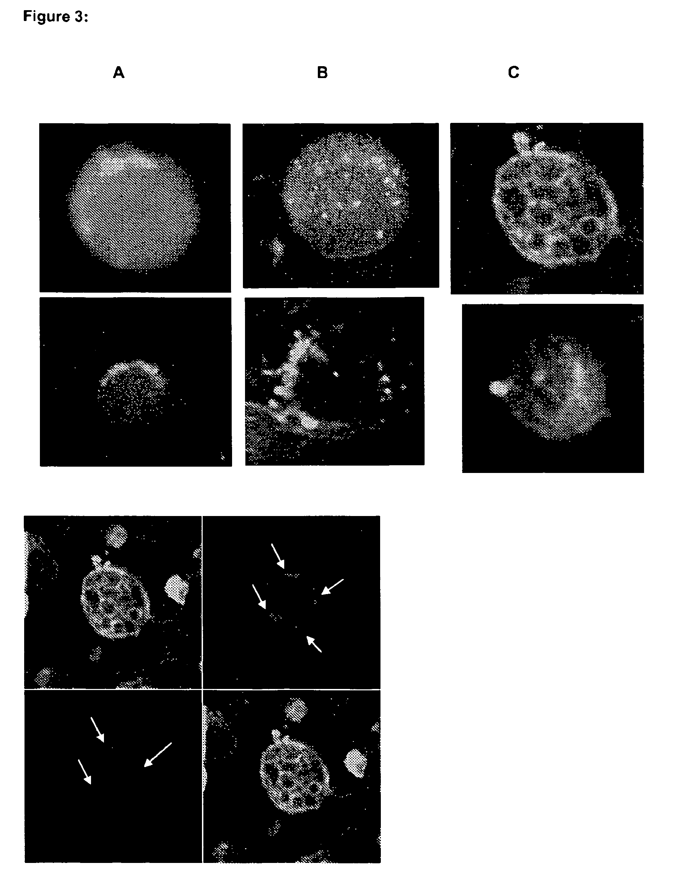

### Figure 3. TLR2 and mCAT1 colocalize in microrafts upon virus transduction

B cells were labeled by incubation with FITC-cholera toxin (FITC-CTx, green fluorescence) for sphingolipids, anti-TLR2 antibody (red) and anti-mCAT1 antibody (blue). The upper pictures show patterns obtained with antibodies and lower pictures patterns resulting from cross-reacting sphingolipids by addition of an antibody to CTx. Columns A, B and C show resting B cells, LPS-activated B cells and B cells activated by LPS and transduced, respectively. The insert shows the breakdown of the upper right picture into individual staining pattern for sphingolipids, TLR2 (in red with arrows), mCAT1 (in blue with arrows) and the triple staining.

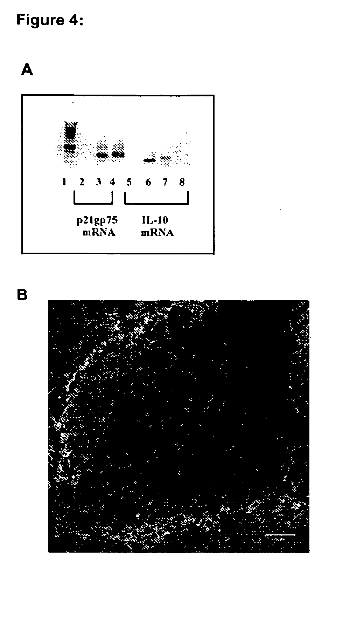

### Figure 4. Transduced B cells localize and remain within the spleen marginal zone for several months

**A**. *Ex vivo* semi quantitative RT-PCR analysis demonstrating the presence of IL-10 and p21 gp75 transcripts in p21 gp75 transduced B cells obtained from the spleen 3 months after transfer of transduced B cells. Lane 1: MW marker (smart ladder); lanes 2&3: Negative and positive controls for plasmid pMND-p21gp75 construct, respectively; lane 4: p21gp75 transduced B cells; lanes 5&6: Negative and positive controls for IL-10, respectively; lane 7: p21 gp75 transduced B cells; lane 8: B cells activated by LPS only. **B.** Spleen as observed by confocal microscopy (Zeiss CLM510; 20x magnitude) 3 days after intraperitoneal transfer of SNARF-1 stained transduced B cells (10-15x10⁶). The white pulp is delineated by the staining of metallophilic macrophages using MOMA-1 (rat IgG2a) recognized by Alexa 488 labeled goat anti-rat IgG antibody (green); SNARF-1 stained cells appear in red. Bar=50µm

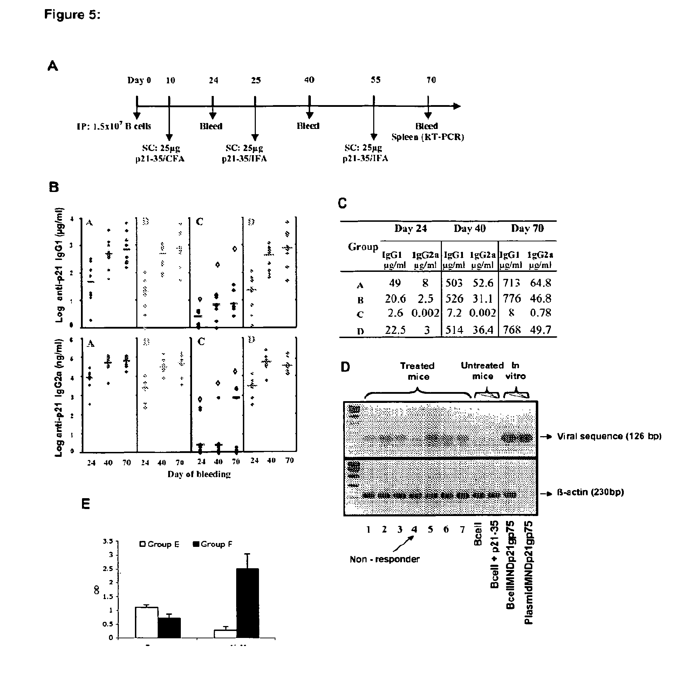

### Figure 5. Prevention of specific antibody response in transduced B cells recipient mice

**A.** Outline of the protocol for B cell adoptive transfer and p21-35 immunization; **B.** Concentrations of anti p21-35 IgG1 and IgG2a antibodies measured by ELISA after each of 3 consecutive immunizations. Group A mice (n=8) were transferred with LPS-activated B cells loaded with p21-35; group B mice (n=8) were transferred with naive LPS-activated B cells and group D mice (n=8) were transferred with GFP-transduced B cells. Group C mice (n=8) were transferred with p21gp75 transduced B cells. The upper panel shows individual anti-p21-35 IgG1 titers (µg/ml) and means after each immunization. The lower panel shows individual anti-p21-35 IgG2a titers (ng/ml) and means; **C.** Median values of anti p21-35 antibodies; **D.** RT-PCR analysis demonstrating the presence of transgenic B cells in recipient mice 3 months after adoptive transfer. mRNA was extracted from individual mouse spleen cells (n=7) adoptively transferred with transduced B cells and immunized with p21-35 (group C). As a control, mRNA was extracted from B cells freshly transduced with the p21 gp75 vector. RT-PCR products were separated by electrophoresis on a 1.5% agarose gel and the size of the products was compared to that of plasmid pMND-p21gp75. Lower bands represent amplified ß-actin. A MW marker is shown on the left; **E.** p21-35 and tetanus toxoid specific IgG1 antibodies obtained in mice adoptively transferred with p21 gp75 transduced B cells (group E; n=4) and in control naive mice (group F; n=4). Mice were immunized simultaneously by SC injections of p21-35 and tetanus toxoid in IFA. Data are presented as medians ± SEM.

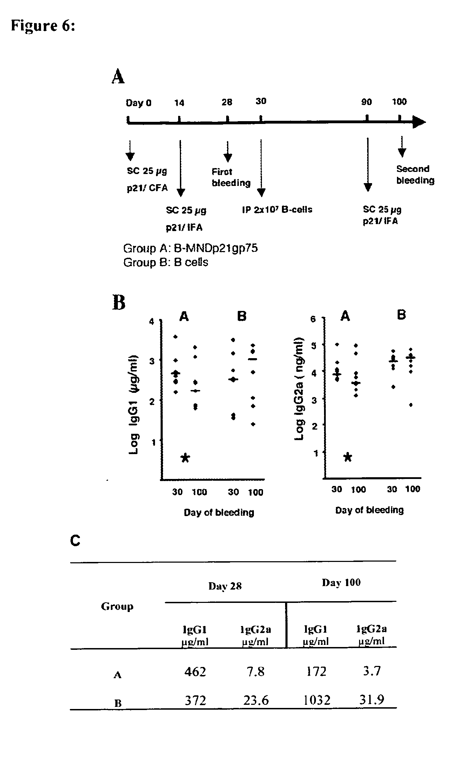

### Figure 6. Suppression of specific antibody response in transduced B cells recipient mice immunized with p21-35

**A.** Outline of the protocol for p21-35 immunization and B cell adoptive transfer; **B.** Concentrations of anti p21-35 IgG1 and IgG2a antibodies measured by ELISA after two immunizations and prior to cell transfer, and after the third immunization. Group A mice (n=8) were transferred with p21gp75 transduced B cells and group B mice (n=8) were transferred with naive LPS-activated B cells. Individual values and medians of anti p21-35 IgG1 titers (left; µg/ml) and IgG2a (right; ng/ml) obtained after 2 and 3 immunizations are shown; **C**. Median values of anti p21-35 antibodies.

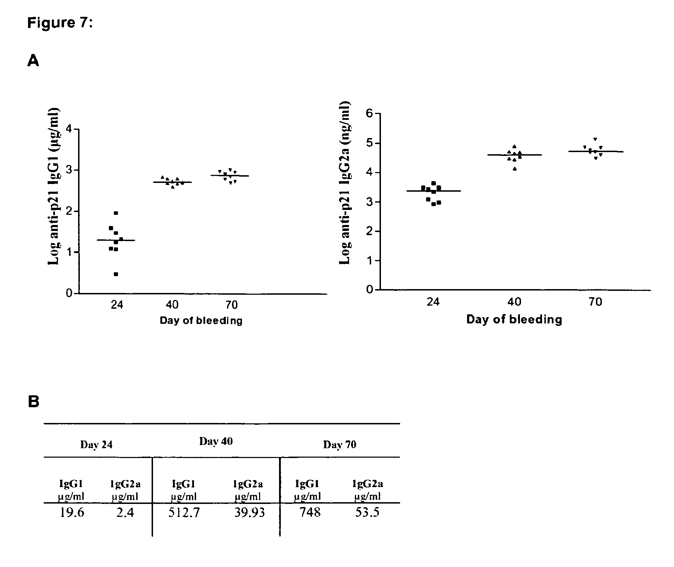

### Figure 7. p21gp75 transduced B cells from IL-10-/- mice do not confer specific unresponsiveness

**A.** BALB/c mice were transferred with LPS-activated p21gp75 transduced B cells from IL-10 deficient mice. Concentrations of anti p21-35 IgG1 and IgG2a antibodies were measured by ELISA 2 weeks after each of 3 consecutive immunizations. The left panel shows individual anti-p21-35 IgG1 titers (µg/ml) and means after each immunization. The right panel shows individual anti-p21-35 IgG2a titers (ng/ml) and means. **B.** Median values of anti p21-35 antibodies.

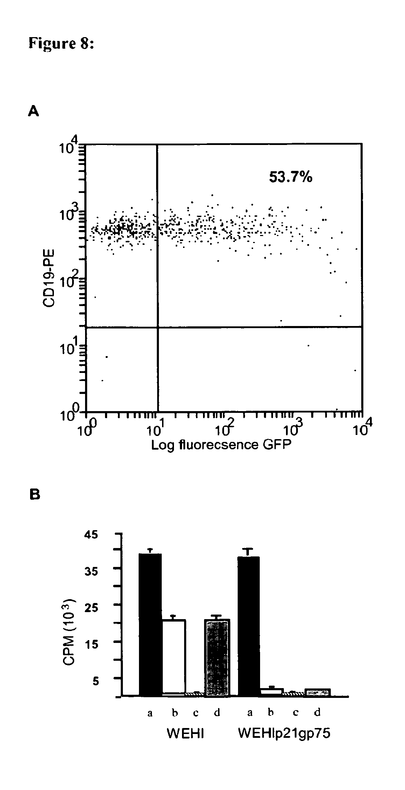

### Figure 8. B cells are efficiently transduced and present p21-35 by MHC class II determinants

**A.** FACS analyses of CD19 positive cells activated by LPS and transduced by 2 runs of centrifugation with the MND-GFP vector. The percentage of GFP-positive B cells is illustrated as obtained 24h after transduction; **B.** Lysis of non-irradiated WEHI cells (left) or WEHI cells transduced with the p21gp75 vector (right) in the presence of a p21-35 specific ctotoxic T cell line (G-121). Thymidine incorporation (CPM) was used as a parameter reflecting cell lysis. WEHI cells were incubated alone (v; a), in the presence of the G-121 T cell line without added p21-35 peptide (□;b), with the G-121 T cell line and addition of p21-35 (dotted column; c), or with the G-121 T cell line and addition of a control p71-85 peptide (striped column, d). Peptides were added at 18 µM. Co-cultures were seeded at 1/1 target/effector ratio and harvested 32 hours later. Data shown are means and SD from triplicate wells.

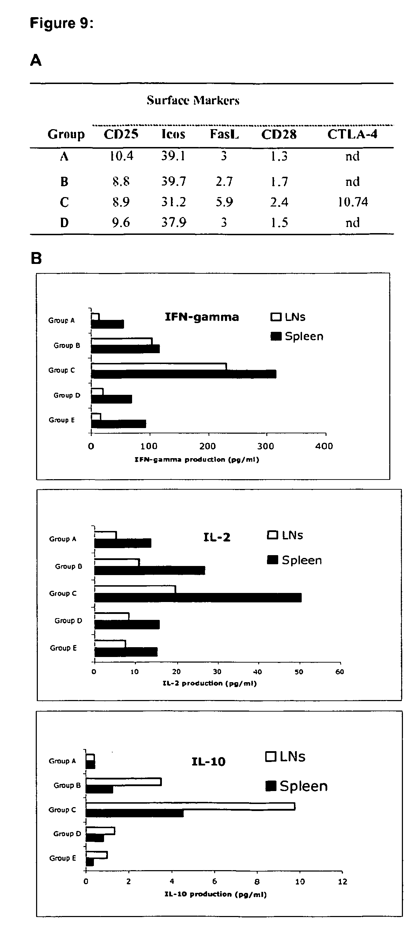

### Figure 9. Recipients of p21-35 transduced B cells show T cell expressing CTLA-4 and producing IFN-γ and IL-10 after p21-35 immunization

**A.** BALB/c mice were adoptively transferred with LPS-activated B cells either loaded with p21-35 (group A), with no peptide (group B), transduced with p21-35 (group C) or transduced with GFP (group D). CD4⁺ spleen T cells obtained 10 days after B cell transfer and p21-35 injection were analyzed by FACS for expression of 5 activation markers. Figures represent percentages of positive cells in pools of 4 spleens. nd: not detected; **B.** IFN-γ, IL-2 and IL-10 production by spleen and lymph node cells (pools of 4 mice for lymph nodes) measured by ELISA in supernatants of cells incubated with 30µM p21-35 for 48h. Mouse groups are as in (A). A group of mice not receiving B cells was added as a control (group E). Results are given in pg/ml.

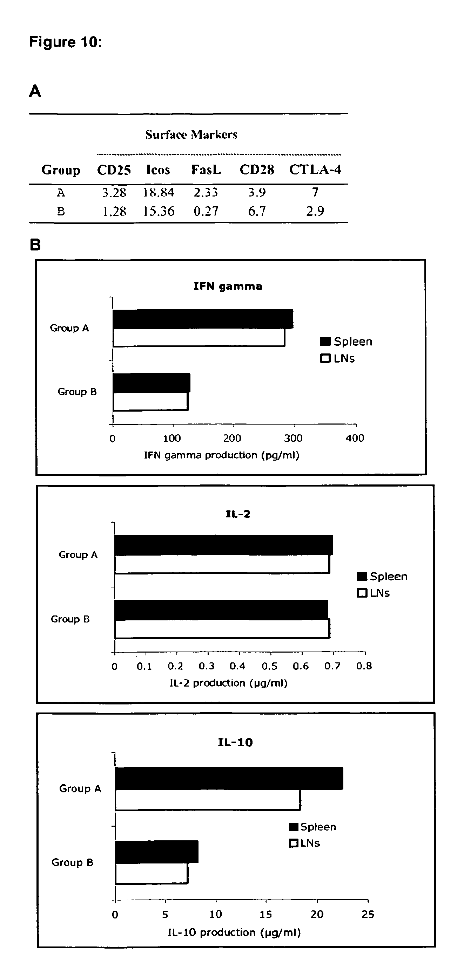

### Figure 10. p21-35 immunized recipients of transduced B cells show increased CTLA-4 expression by T cells and production of IFN- and IL-10

**A.** BALB/c mice were immunized by SC administration of p21-35 followed by adoptive transfer of either LPS-activated p21-transduced B cells (group A) or B cells activated by LPS (group B). CD4⁺ spleen T cells obtained 10 days after cell transfer were analyzed by FACS for expression of 5 activation markers. Figures represent percentages of positive cells in pools of 4 spleens; **B.** IFN-γ, IL-2 and IL-10 production by spleen and lymph node cells (pool of 4 mice for lymph nodes) was measured by ELISA in supernatants of cells incubated with 30µM p21-35 for 48h. Mouse groups are as in Figure 5. Results are given in pg/ml.

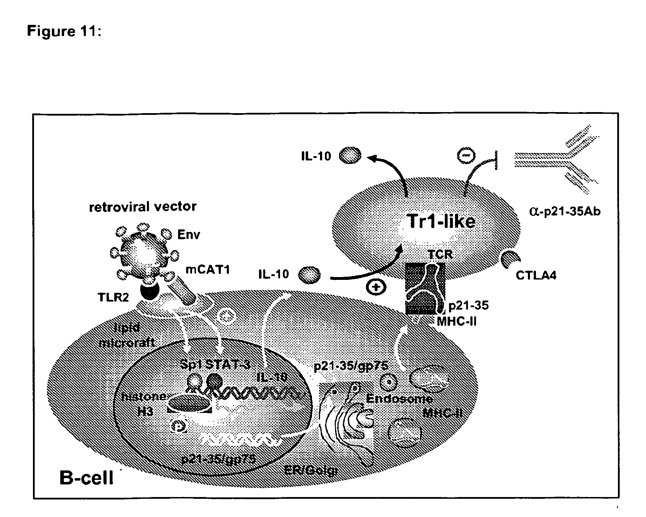

### Figure 11. Summary sketch of the mechanism by which retrovirally transduced B cells induce antigen-specific unresponsiveness

### EXAMPLES

### Materials and methods to the examples

### B cell transduction

Murine B lymphocytes were obtained from spleens of naïve BALB/c mice by Ficoll density gradient purification (Nycomed Pharma, Oslo, Norway) and subsequently enriched by positive selection using magnetic beads coupled with antibodies to CD19 (Miltenyi Biotec, Bergisch Gladbach, Germany). Purified B lymphocytes were cultured in the presence of 50 µg/ml LPS in enriched RPMI medium (Life Technologies, Breda, The Netherlands), containing 10% FCS, 50µM 2-mercaptoethanol, 100 U/ml penicillin and 100 µg/ml streptomycin. The next day, concentrated viral vector-containing supernatant (200µ1; preparation in supplemental data) supplemented with 6-µg/ml polybrene was added to the cells. To enhance transduction efficiency, cells were subjected to two successive rounds of centrifugation with vector-containing medium for 1 hr at 2600 rpm and 20°C, separated by a 4 hr interval. GFP-transduced B cells were stained with anti-mouse CD19 PE-conjugated monoclonal antibodies (BD Biosciences, San Diego, CA). Isotype-matched PE-conjugated mAb were used as negative controls. After staining and washing, 10,000 events were acquired with a FACSCalibur and analyzed by CellQuest software (BD Biosciences). Dying cells were excluded by gating and GFP fluorescence was measured to assess gene transfer efficiency. B cell survival was monitored by propidium iodide staining and cytofluorimetric analysis. All results were triplicates of independent samples and significance was calculated by one-way analysis of variance (ANOVA).

### Phenotypic analysis of transduced B cells

Transduced B cells were tested for cell surface marker expression by direct immunofluorescence and flow cytometric analysis using antibodies conjugated to either FITC or PE (BD Biosciences). Aliquots of cells were incubated with specific antibodies to either CD19-FITC or CD-19-PE and CD40 (PE; clone 3/23), CD80 (PE; clone 16-10AI), CD54 (PE; clone 3E2), CD86 (PE; clone GL1), IA/IE (FITC; clone 2G9), CD45R/B (FITC; clone 16A). Isotype controls were included. IL-10 was measured in cell supernatant of transduced versus non-transduced B cells using the BD OptEIA^{™} Set Mouse IL-10 (BD Biosciences). Intracellular IL-10 was determined using IL-10-specific antibody (clone: IES5-16E3; PE-labeled; BD Biosciences) on the CD19⁺ gated population. Non-specific binding was evaluated with isotype controls. mRNA for IL-10 was purified using the QuickPrep Micro mRNA purification kit (Amersham Biosciences, Buckinghamshire, UK). IL-10 cDNA was synthesized using the First Strand cDNA synthesis kit (Amersham Biosciences) and PCR carried out using Human/mouse IL-10 PCR primer pair (R&D systems Europe, Abingdon, United Kingdom). Mouse TH1/TH2 set 1 Multiplex PCR^{©} kit (BioSource Europe, Nivelles, Belgium) was used for detecting IL-10, IFN-γ, IL-2, IL-12p40, IL-4 and IL-13 gene expression.

To investigate the role of Toll-like receptors in induction of immune unresponsiveness, blocking studies were carried out. CD19 purified B cells were activated overnight by LPS and co-cultured with 1µg/10⁶ cells antibodies to either TLR-2, TLR-3, CD180 or an IgG2a isotype control, respectively, for 30 minutes at 37° prior to each of the two runs used for transduction. Blocking antibodies to TLRs (clone 6C2 to mouse TLR2 and clone TLR3.7 to human TLR3), an anti-mouse CD180 (clone MHR73-11) or with an IgG2a isotype control (clone eBM2a) were purchased from BD Bioscience. Clone TLR3.7 was shown to cross-react with mouse TLR3.

### MHC-II presentation of the p21gp75 antigen

To validate that the p21 gp75 fusion peptide is presented in association with MHC-II, a proliferation assay was conducted using WEHI 231 cells transduced with the MND-p21gp75, and an MHC-II restricted cytotoxic T cell line (G-121) specific to p21-35 (Janssens et al., 2003a). WEHI 231 cells were seeded in 6-well plates (5x10⁵ WEHI cells/ml) in D10 medium and incubated for 3 consecutive periods of 12 hours with the MND-p21gp75 vector supplemented with polybrene (6 µg/ml). WEHI cells stably transduced with the MND-p21gp75 vector were obtained by selection in G418 (800 µg/ml). Transduced and non-transduced WEHI cells were then co-cultured for 32 hr with the MHC-II restricted cytotoxic T cell clone G-121 at a 1:1 ratio (5x10⁴ cells/well). The G-121 clone did not proliferate under those conditions (Janssens et al., 2003a). In some conditions, 15-mer-peptides were added corresponding to peptides p21-35 and p71-85. Proliferation was evaluated by measuring thymidine incorporation, following addition of 1 µCi/well of [³H]thymidine during the last 18 hr of incubation. Each experiment was performed in triplicate, and results are expressed as mean ± SD.

### In vivo studies

For prevention experiments, B cells stably transduced with the MND-p21gp75 vector (1.5x10⁷/mouse) were injected intraperitoneally (IP) into naive 6-8 week-old female BALB/c mice. Ten days after cell transfer, recipient mice were challenged with a subcutaneous (SC) immunization of p21-35 (25µg/100µl) in complete Freund's adjuvant (CFA) and boosted 14 days later with the same dose in incomplete Freund's adjuvant (IFA). Ten days after the second immunization, the animals were bled and sera were assessed for p21-35 specific antibodies (IgG1 and IgG2a) by ELISA. Three control groups were added: a group of mice received B cells activated with LPS and loaded with p21-35 by overnight incubation with soluble peptide (30 µg/ml); a second group of mice received B cells only activated by LPS during 24 hours; and a third group received LPS activated B cells transduced with the MND-GFP control vector. All mice belonging to the control groups were submitted to the same immunization protocol with p21-35. For suppression experiments, an immune response was first established by 2 consecutive immunizations with p21-35 (25 µg/mouse) in CFA/IFA. Successful immunization was confirmed at day 28 by assaying anti-p21 antibody titers. At day 30, mice were randomly separated into two groups (7 mice/group); group A was injected IP with 2x10⁷ LPS activated B cells transduced with the MND-P21gp75 and the control group B received 2x10⁷ LPS activated but not transduced B cells. The anti-p21-35 antibody titers were determined by ELISA. All experiments were carried out in accordance with rules established by the University Ethical Committee for animal experiments.

### Expression of p21gp⁷⁵ in transduced B-cells in vivo and splenic distribution

Expression from the p21 gp75 construct in the transduced B cells in vivo was analyzed by reverse transcriptase (RT) PCR analysis. Three months after adoptive transfer, mice were sacrificed and B cells were purified from spleen by Ficoll density gradient purification and enriched by positive selection using magnetic beads coupled with antibodies to CD19. mRNA was extracted using the QuickPrep Micro mRNA purification kit (Amersham Biosciences) and converted into cDNA using the First Strand cDNA synthesis kit (Amersham Biosciences). PCR was carried on cDNA using retroviral vector-specific primers, 5'-CCCTTTATCCAGCCCTCACTC-3' and 5'CCTGGGGACTTTCCACACCC-3'. RT-PCR and PCR products were separated by gel electrophoresis and visualized with a Stratagene Eagle Eye II (Stratagene, La Jolla, CA).

To confirm presence of the transduced B cells in the marginal zone of the white pulp of the spleen, SNARF-1 (Molecular Probes, Eugene, OR) labeling for B cells and rat anti mouse metallophilic macrophages monoclonal antibody (MOMA-1; Serotec Ltd, Oxford, UK) were used. Transduced B cells were washed twice in PBS and resuspended for 15 min at 37°C at a density of 107 cells/ml in PBS containing 12.5nM of the succinimidyl ester of SNARF-1 carboxylic acid acetate. Splenic distribution of transferred B cells was analyzed on 10-µm splenic cryostat sections fixed in acetone and stained with MOMA-1 and Alexa fluor® 488 labeled goat anti-rat IgG (H+L) antibody (Molecular Probes, Eugene, OR). Immunofluorescence slides were mounted in prolong® gold antifade reagent (Invitrogen, Eugene, OR) and analyzed with a Zeiss CLM510 confocal microscope using excitation between 488 nm and 530 nm while monitoring fluorescence emission at two wavelengths, typically 580 nm and 640 nm.

### Detection of TLR2, mCAT1 and lipid rafts

B cells were incubated with 8 µg/ml FITC-conjugated CTx B (Sigma aldrich) on ice for 10 min. After fixation, staining was carried out with antibodies to the mouse toll like receptor-2 (TLR2; clone: mT2.7; e-Bioscience) and to CAT-1 (the goat polyclonal IgG antibody; clone: c-12; Sigma). After washing, cell suspensions were incubated with Alexa 594-coupled second antibody for retroviral cellular receptor mCAT-1 for 1 hour. Cell fluorescence intensity was analyzed with a Zeiss CLM510 confocal microscope.

### Western blotting

Western blotting was performed using SP1 (mouse monoclonal IgG1; clone: 1C6; Santacruz Biotechnology) and Phospho-STAT3 (Tyr705) antibody (Cell Signaling Technology) on whole B cell lysates.

### Chromatin immunoprecipitation (Chip assay)

Anti-Acetyl-Histone H3 (Lys 14; rabbit antiserum) and Anti-Phospho-Histone H3 (Ser 10; rabbit monoclonal IgG; clone: MC463) were purchased from Millipore (Billeria, USA). ChIP assays were performed following ultrasonic shearing conditions that result in DNA fragment size of -300 bp. Remaining procedures were conducted as previously described (Lucas et al., 2005). The primers used to amplify specific regions of the IL-10 promoter (STAT3) by RT-PCR are 3'TCA-TGC-TGG-GAT-CTG-AGC-TTC-T5' and 5'CGG-AAG-TCA-CCT-TAG-CAC-TCA-GT3'.

### Statistical analysis

Statistical evaluation was carried out with the Instat 3 software (GraphPad Software Inc., San Diego, CA). Triplicates of FACS, thymidine incorporation and thymidine release results were expressed as mean with SD. Differences in antibody titers were evaluated by Kruskal Wallis, Mann-Whitney or paired Wilcoxon test, where appropriate. For B-cell transduction efficiency and 3T3-titers, significance was calculated by unpaired Student's t-test. A two-sided p-value ≤0.05 was considered as significant.

### Animals and reagents

BALB/c (H-2^{d}) mice were obtained from the University animal facilities. B6.129P2-II10^{tm1Cgn}/Crl IL-10 deficient mice (backcrossed for 18 generations on the BALB/cJ background) were purchased from Charles River Laboratories (Wilmington, MA). Smooth lipopolysaccharide from *Escherichia coli* (055:B5) was purchased from Sigma (Steinheim, Germany). Smartladder and 15-mer-peptide p21-35 derived from the Der p 2 sequence was purchased from Eurogentec (Liège, Belgium). Peptide sequences were CHGSEPCIIHRGKPF and NACHYMKCPLVKGQQ, respectively, with a purity>85%. TaqMan probe (5'-CCG-GCC-GCT-TGG-GTG-GAG-AG) and primers set for NeoR, forward primer (GAT-GGA-TTG-CAC-GCA-GGT-T) and reverse primer (GTG-CCC-AGT-CAT-AGC-CGA-ATA) were also purchased from Eurogentec. WEHI 231-cells were purchased from the European collection of cell cultures (ECACC, Salisbury, UK).

### Retroviral vector generation

The MLV retroviral vector plasmid pMND-GFP was generated by cloning the GFP gene into the pMND-MFG-SN vector (kindly provided by Dr. D. Kohn, Children's Hospital of Los Angeles, USA). The chimeric construct p21gp75, encoding peptide 21-35 connected to the transmembrane and cytosolic part of gp75 (amino acid 488-539) via a linker Ser-Gly-Gly-Ser-Gly-Gly-Ser-Gly-Gly, was made by PCR using partially overlapping primers (5'-CCGGAATTCCCACCATGGATTGCCATGGTCAGAACCATGTATCAT TCATCGTGGTAAACCATTCTCTGGTGGTTCCGGAGGTTCAGGAGGC-3', 5'-CAGACAAGAAGCAACCCCGAAAATGGCAGCTACAAGTAACAACGCAGCCAC TACAGCAATGGTAATGATGCCTCCTGAACCTCCGGAACC-3', 5'-GCATAGCGT TGATAGTGATCAGTGAGGAGAGGCTGGTTGGCTTCATTCTTGGTGCTTCTAGA ACGGATCAGACAAGAAGCAACCCCG-3' and 5'-GTATCTCTCGAGTCAGACCAT GGAGTGGTTAGGATTCGGGAGCTCCTCATAGTCCTCAGCATAGCGTTGATAGTGATCAG-3'). The pMND-p21 gp75 retroviral vector was generated by restricting the p21 gp75 fragment with EcoR I/Xho I and subsequent cloning into the respective sites of pMND-GFP. Stable retroviral vector producer cell lines that produced the MND-GFP and MND-p21gp75 retroviral vector particles were obtained by introducing the corresponding constructs into GPE86 (Markowitz et al., 1988) packaging cells and subsequent selection in G418 (800 µg/ml), as described previously (Janssens et al., 2003), The resulting packaging cell lines, designated as GPE-MND-GFP and GPE-MND-p21 gp75, were grown in Dulbecco's modified Eagle's medium (Gibco-BRL, Grand Island, NY) supplemented with 10% heat-inactivated fetal bovine serum (Gibco-BRL), 100U/ml penicillin, 100µg/ml streptomycin and 200mM L-glutamine (designated as D10 medium). Conditioned medium was collected over 24 hr from confluent vector producer cell lines. This vector-containing supernatant was concentrated 200-fold using centricon ultrafiltration (Millipore Corporation, Bedford, MA). By using quantitative real-time PCR with TaqMan's set of specific primers and the probe for the amplification of neaR gene (Venditti et al., 2003), vector genome copies in concentrated supernatants of packaging cell lines for the GFP and p21gp75 construct were established at 9x10⁹ and 3.9x10¹⁰ per ml, respectively.

### Antibody responses

To determine the anti-p21-35 antibody titers, neutravidin microtitration plates (Perbio Science, Erembodegem, Belgium) were coated with biotin-labeled p21-35 (2µg/ml in PBS, 100 µl) by overnight incubation at 4°C. Plates were washed with PBS-0.05% Tween (PBST) and residual binding sites were blocked by 100 µl of PBS-0.5% BSA for 1 hour at RT. Fifty µl of serially diluted sera (1/200, 1/1000, 1/5000) were then added to the plates and incubated for 2 hours at RT. Specific antibody binding was detected by addition of 50µl peroxidase-conjugated goat anti-mouse antibody (BD Biosciences) to either IgG2a or IgG1 and o-phenylenediamine (OPD). Anti-p21-35 IgG1 and IgG2a antibodies were titrated against a standard curve established with a specific mAb of the same isotype produced in our laboratory. As control, microtitration plates (MaxiSorp™Surface; NUNC, Denmark) were incubated at 4°C overnight with 100µl PBS containing 5µg/ml of tetanus toxoid (Aventis Pasteur, Marcy l'Etoile, France). Specific antibody binding was detected as described for p21-35 specific antibodies.

### T cell activation

T cells were obtained by Ficoll density gradient purification of splenocytes and negative selection (purity 85-90%) using magnetic beads against CD11c, CD11b and CD45R (Miltenyi Biotec; Bergisch Gladbach, Germany). Pools of T cells from 4 mice were tested for cell surface antigen expression by direct immunofluorescence and flow cytometric analysis using antibodies to CD25 (PE; clone 7D4), CD28 (PE; clone 37.51), ICOS (PE; clone 7E.17G9), CTLA-4 (PE; clone UC10-4F10-11), IA/IE (FITC; clone 2Gg), all antibodies obtained from BD Biosciences. T cells collected from lymph nodes and spleen 10 days after the second immunization were cocultured (10⁶ cells/200 µl) in flat-bottomed 96-well plates with peptide (p21-35, 30 µM). Antigen presenting cells (APC) were prepared by total B and T cell depletion from splenocytes and lymph node cells with CD19 and CD90 beads, respectively (Miltenyi Biotec). Supernatants were collected 24 and 48 hours later for cytokine assay. IFN-γ, IL-2 and IL-10 were assayed using the BD OptEIA™ Set Mouse (BD Biosciences).

### Example 1: B cells are efficiently transduced for p21-35 presentation by MHC class II determinants

B cells prepared from splenocytes of naïve BALB/c mice were activated with LPS before transduction with a Moloney murine leukemia virus-based retroviral vector encoding the Der p 2-derived T cell epitope p21-35 fused to the gp75 protein (MND-p21gp75). We used a GFP control vector (MND-GFP) to determine the transduction efficiency under the same experimental conditions as for the MND-p21gp75 vector. Both the MND-GFP and MND-p21gp75 vectors had similar particle titers, that typically fell in the range of 10¹⁰- 4x10¹⁰ genome copies (gc)/ml. Both GFP and p21gp75 were driven from the same modified long terminal repeat promoter (MND-LTR (Halene et al., 1999). The vectors also contained an SV40-Neo^{R} expression cassette. Figure 8A shows that 200µl of a highly concentrated vector preparation applied to LPS-activated B cells resulted in more than 50% transduction efficiency. We then determined whether the p21-35 peptide was actually presented within the context of MHC-class II determinants. To this end, we took advantage of the cytotoxicity of a T cell line (G-121) known to lyse target cells after cognate interaction with p21-35 (Janssens et al., 2003a). The WEHI-231 B cell line (WEHI) was first stably transduced with the MND-p21gp75 vector (WEHI-p21gp75), as confirmed by PCR (data not shown), and subsequently selected in neomycin. Transduced WEHI cells were co-cultured with the G-121 T cell line for 32h. As the T cell line did not proliferate under such conditions (Janssens et al., 2003a), thymidine incorporation was used as a readout of WEHI cell lysis. WEHI-p21gp75 cells were induced into apoptosis by the G-121 T cell line in the absence of added soluble p21-35, indicating that transduced WEHI cells were capable of presenting the intracellularly expressed p21-35 T cell epitope in the context of MHC class II molecules (Figure 1 B). In contrast, non-transduced WEHI control cells were induced into apoptosis by the G-121 T cell line only after addition of soluble p21-35 peptide (Figure 8B). In contrast, non-transduced WEHI control cells only failed to proliferate in the presence of the G-121 T cell line after addition of soluble p21-35 peptide (Figure 8B).

Retroviral transduction of B cells could alter cell phenotype, with possible consequences on the mechanism by which such cells could induce antigen unresponsiveness in vivo. We therefore compared the expression of five surface activation markers by FACS analysis of resting B cells, LPS-activated and transduced B cells, and B cells only activated by LPS, respectively. LPS activation up-regulated CD40 (60% of cells) and CD86 (57%) but not CD80, MHC-class II or CD54. Transduction further increased expression of CD40 and CD86, with 97 and 93% of transduced cells rendered positive, respectively.

### Example 2: B cell transduction results in constitutive expression of IL-10 following colocalization of TLR2 and mCAT1 at cell surface

LPS activation of B cells induces transient production of IL-10 (Burke et al., 2004). We tested the supernatants of B cells for the presence of IL-10 24 h after transduction using ELISA. As expected IL-10 was detectable in supernatants of transduced and non-transduced of B cells activated by LPS (2295 pg/ml), as well as in cells incubated with polybrene but no viral particles. Surprisingly, however, supernatants of MND-GFP transduced (3889 +/- 184 pg/ml) or MND-p21gp75 transduced B cells (4149 +/- 114 pg/ml) showed twice as much IL-10 compared to non-transduced, LPS-activated B cells (2295 pg/ml), We subsequently confirmed by intracellular staining using an anti-IL-10 antibody and we could confirm that 30% of actually transduced B cells (versus 11 % of LPS activated cells) contained detectable IL-10, with a fluorescence intensity 2.5-fold higher than B cells only activated by LPS (Figure 1A).

These findings suggested that retroviral transduction increased IL-10 transcription. The latter depends on activation of STAT-3 and/or SP1 transcription factors (Brightbill et al., 2000; Riley et al., 1999).Western blot analysis showed that phosphorylated STAT-3 and Sp1 were induced in transduced LPS activated B cells, In contrast, only low level phosphorylated STAT-3 and no SP1 could be detected in non-transduced LPS activated B cells (Figure 1B).

Histone 3 phosphorylation is associated with increased accessibility of binding sites for STAT-3 and SP1 (Zhang et al., 2006). We therefore evaluated changes in histone H3 phosphorylation across the IL-10 gene in transduced LPS activated B cells. To this end, we generated chromatin fragments of 300 bp average sizes, which allowed high resolution profiling of histone modifications across the first 1,600 bases of the IL-10 promoter. A comparison was run between resting, LPS activated, and transduced B cells for phosphorylation at Ser10 using the Chromatin immunoprecipitation (ChIP) assay. A substantial increase in phosphorylated histone 3 binding was noted at that STAT-3 binding site in transduced LPS-activated B-cells compared to non-transduced, LPS-activated or unstimulated B-cells (Figure 1C). Hence, retroviral transduction of B cells resulted in epigenetic imprinting at the level of binding sites for IL-10 transcription factors, particularly STAT-3.

This effect seemingly resulted from the initial contact between the viral vector particle and the cell surface. This is likely mediated by the interaction of the retroviral envelope protein (Env) with its cognate cellular receptor, mCAT-1 (Masuda et al., 1999). As, the SU-domain of the viral envelope (Env) is a glycoprotein (Panda et al., 2000) and since peptidoglycans, such as those of *Candida albicans* production (Netea et al., 2004), have been reported to activate Toll-like receptor 2, which resulted in IL-10, we hypothesized that the retroviral envelope could partially activate TLR2. We tested this hypothesis by using blocking antibodies to TLR2 or TLR3 (as a control) prior to and during transduction. The production of IL-10 and its transcription as assessed by RT-PCR were completely abolished when transduced B cells were incubated with a TLR2-specific blocking antibody, but not with a TLR3-specific antibody, an anti-CD180 antibody or isotype-specific control antibody (Figures 2A and 2B).

This confirms that retroviral transduction triggers TLR2-mediated induction of IL-10 production. This induction was likely due to the viral vector particles themselves rather than to the intracellular genomic viral vector RNA or cDNA and was independent of reverse transcription or genomic integration. Indeed, exposure of LPS-activated B cells to "empty" retroviral vector particles, that are devoid of viral genomes, also resulted in a significantly increased IL-10 production. Interestingly, TLR2-specific blocking antibodies abrogated both viral vector-induced and LPS-induced IL-10 production. Whether this indicated that in such a system full TLR2 signaling is required for TLR4-induced IL-10 production is an intriguing possibility. An alternative explanation could be found in the recent demonstration that commercially available LPS contained TLR2 ligands in addition to the canonical TLR4 activation ligand.

To directly demonstrate a possible physical interaction between TLR2 and mCAT1, we subsequently evaluated whether the virus receptor mCAT1 was recruited into microrafts together with TLR2. To this end, we made use of FITC-labeled cholera toxin for its capacity to bind to sphingolipids (GM1), together with mCAT-specific and TLR2-specific antibodies. Figure 3 shows resting B cells and cells that were stimulated with either LPS alone, or LPS and viral vector after incubation with FITC-cholera toxin (CTx), anti-mCAT1 and anti-TLR-2 antibodies. In resting cells, the distribution of GM1 on the plasma membrane was homogeneous with diffuse TLR2, both in the membrane and the intracellular compartment and retroviral cellular receptor mCAT-1 was not observed (Figure 3; panel A). In LPS-activated and retrovirally-transduced B cells, translocation of TLR2 to the membrane rafts and retroviral cellular receptor mCAT-1 expression were both significantly increased (Figure 3; panel C). Cross-linking of microrafts by addition of an anti-cholera toxin antibody showed that both TLR2 and mCAT1 cocapped in association with microrafts. Addition of a microraft formation inhibitor, methyl-betacyclodextrin, fully prevented mCAT1 and TLR2 surface expression.

These results suggest that the TLR2-dependent signaling leading to constitutive expression of IL-10 was triggered by co-localization of TLR2 with the mCAT1 receptor in microrafts following its interaction with the viral envelope presumably the SU glycoprotein.

### Example 3: p21gp75 transduced B cells persist for at least 3 months in the spleen

The experiments described above indicated that B cells constitutively produced IL-10 upon retroviral transduction. To determine whether this effect was sustained, and not transitory as for LPS (Gieni et al., 1997), a group of three naive BALB/c mice were adoptively transferred with transduced B cells (1.5x10⁷ cells/mouse were injected IP). A control group received LPS-activated B cells with no transduction. Three months later, spleen B cells were isolated and semi-quantitative PCR analysis revealed that cells expressed both p21-35gp75 transgene encoded by retroviral vector and IL-10 (Figure 4A), thereby establishing that retroviral vector transduction resulted in stable IL-10 transcription. Using the same method, we found no evidence of transcripts for IFN-γ, IL-2, IL-12p40, IL-4 or IL-13:

We then wanted to determine whether B cells localized at places where they could interact with T cells. We therefore adoptively transferred naive BALB/c mice with SNARF-1-labeled transduced B cells by IP injection. Three days later the spleen was removed and frozen spleen tissue sections were fixed and stained with MOMA-1 antibody, a white pulp marker. Tissue sections (Figure 4B) analyzed by fluorescence microscopy to locate adoptively transferred B cells relative to the ring of MOMA-1 positive cells showed the localization of SNARF-1 labeled B cells (red) within the white pulp (green).

### Example 4: p21gp75 transduced B cells prevent antibody production upon peptide immunization

The above results suggest that transfer of transduced B cells could lead to long-term antigen unresponsiveness. This was tested in both prevention and suppression settings.

The p21-35 sequence contains a major B cell epitope in addition to the immunodominant T cell epitope (Wu et al., 2000) and could therefore be used for assessing the effect of transduced B cells on specific antibody production. To evaluate whether p21 gp75 transduced B cells would prevent an immune response towards p21-35, 1.5x10⁷ cells/mouse were injected IP to groups of BALB/c mice (n=8), followed by three SC injections of p21-35 in CFA/IFA. Figure 5A outlines the experimental protocol. In addition to the two groups of mice adoptively transferred with B cells activated with the LPS (group B) and a group receiving p21gp75 transduced B cells (group C) in which persistence of the p21-35gp75 transcript was assessed, a group of 8 mice was adoptively transferred with B cells loaded with the p21-35 peptide (group A) and another group received GFP-transduced B cells (group D).

Figure 5B shows the results of specific IgG1 and IgG2a antibody titers in the four groups of mice. Median antibody values are given in Figure 5C. An increase in p21-35 specific IgG1 and IgG2a antibodies was observed following each immunization in mice having received LPS-activated non-transduced B cells with (group A) or without loaded peptide (group B), or GFP-transduced B cells (group D). In fact, antibody concentrations at the time of first bleeding (day 24) were significantly higher in group A mice (B cells loaded with p21-35) than in group B or D (Kruskal-Wallis test, p<0.0003 for IgG1 and for IgG2a). By contrast, mice receiving B cells transduced with the p21gp75 construct (Group C) showed much reduced values (at days 24, 40 and 70) for both IgG1 and IgG2a antibodies (Kruskal-Wallis test, p<0.002 for IgG1 and p<0.0008 for IgG2a as compared to each of the other groups). Yet in vitro, peptide-loaded and transduced B cells presented the peptide with comparable efficacy as evaluated by p21-35 specific T cell activation.

RT-PCR analysis revealed that the stably integrated vector genomes stably expressed the p21-35gp75 mRNA for at least 3 months post-injection of the genetically modified B cells in 6 out of 7 mice (Figure 5D). Only one mouse (n°4, Figure 5D) failed to express p21-35gp75, which explains why no reduction in antibody titer was apparent (open symbols in Figure 5B, column C). Moreover, this mouse failed to express the IL-10 transcript. These data suggest that the gene-modified B cells may not have engrafted in that mouse and hereby further establish a causal relationship between long-term persistence of gene-modified B cells and lasting tolerance induction. These observations show that transduced B-lymphocytes expressing the T cell epitope of p21-35 in MHC class Il determinants effectively prevented a specific immune response even after recurring peptide injections with a potent adjuvant. Peptide-loaded and B cells transduced with MND-p21 gp75 presented the peptide with comparable efficacy as evaluated by p21-35 specific T cell activation.

To verify that unresponsiveness to immunization was specific for the peptide, an additional group of 4 mice was treated according to the same protocol except for co-immunization with tetanus toxoid (50 µg in 100 µl). Titers of both p21-35 and tetanus toxoid antibodies were evaluated and compared to those obtained in naïve mice. Figure 5E shows IgG1 antibody titers for p21-35 and tetanus toxoid 10 days after immunization. As expected, we found a significant decrease in the titer of anti-p21 IgG1 (Mann-Whitney U test, p<0.03), but no difference in the titer of tetanus toxoid IgG1.

We next determined whether the transfer of transduced B cells affected the antigen-specific T cell response. Four groups of mice were treated according to the protocol described in Figure 5A, but mice were sacrificed 10 days after the second peptide injection. Purified CD4+ T cells were obtained by negative selection from splenocytes and used to assess expression of five surface activation markers by FACS analysis. The results are shown in Figure 9A. T cells expressing surface CTLA-4 were observed only in Group C mice, which had been transferred with p21gp75 transduced B cells. A doubling of FasL+ cell numbers was also observed in the same group. No other significant phenotypic alterations were seen. Th1 and Th2 cytokines were evaluated in supernatants of T cells prepared from spleen or peripheral lymph nodes after 48h of culture with peptide. A group of mice that had received no B cells was added (group E) for a control. As shown in Figure 9B, an increased production of IL-2, IL-10 and IFN-y was observed only with cells obtained from animals treated with p21 gp75 transduced B cells (group C).

These data suggest that B cells transduced with the p21 gp75 construct induced activation of type1-like regulatory T cells (Treg1) (Roncarolo et al., 2001).

### Example 5: p21gp75 transduced B cells suppress ongoing specific antibody production

We next evaluated whether transduced B cells also suppressed an established immune response towards the peptide. To this end, BALB/c mice (n=14) were immunized by two SC injections of p21-35 in CFA/IFA. Mice were randomly separated into 2 groups at day 30, just before cell transfer. Group A and B mice received p21gp75 transduced B cells or LPS-activated B cells (2x10⁷ per mouse in each case), respectively (Fig 6A). Thirty days after cell transfer, all mice received a boost injection with p21-35 in IFA. Serum anti-p21-35 antibodies were titrated two weeks later and their titers compared with those obtained prior to B cell transfer. Group B mice showed an increase of specific antibodies after immunization, as expected (Figure 6B and 6C). By contrast, mice from group A had a significant decrease in median anti-p21-35 IgG1 and IgG2a titers after transfer of transduced cells (paired Wilcoxon test, p<0.02 for both antibody isotypes). These observations indicate that transgenic B cells could, at least partly, suppress an established p21-35 immune response, even when potent adjuvants were used.

Cytofluorimetric analysis of T cell surface markers from spleen cells obtained at the end of the experimental protocol showed an increased proportion of cells carrying CTLA-4 and FasL in the group of mice transferred with transduced B cells (Figure 10A), confirming data shown in prevention experiments. The production of IL-2, IL-10, IL-5 and IFN-γ after 48h of culture with p21-35 was determined as described for prevention experiments (see Figure 9B). As shown in Figure 10B, a significant increase in IL-10 and IFN-γ was observed in mice transferred with p21 gp75 transduced B cells, which is again consistent with a Treg1 phenotype.

### Example 6: IL-10-/- transduced B cells do not confer tolerance

The results so far suggested that transduced B cells exerted a long-lasting prevention and suppression effect on specific antibody production via T cells. In addition, T cells showed upregulation of CTLA-4 and FasL expression together with IL-10 production, both in prevention and suppression experiments. To ascertain that IL-10 production was necessary to confer antigen-specific unresponsiveness, splenic B cells from homozygous IL-10 deficient (IL-10-/-) BALB/c mice were transduced with the MNDp21gp75 vector, according to the same protocol as described above for wild type B cells. Expression of p21gp75 in the IL10-/- B cells was confirmed by RT-PCR. Transduced IL10-/- B cells were transferred to naïve wild type BALB/c mice, followed ten days later by a SC immunization of p21-35 (25µg/100µl) in CFA and two boost injections at days 25 and 55 with the same dose of peptide in IFA (see protocol outline in Figure 5A). The production of specific antibodies was then evaluated.

The results illustrated in Figure 7A and 7B show that the titers of anti-p21-35 IgG1 and IgG2a antibodies were not reduced at any time point in mice receiving IL10-/- B cells transduced with the MNDp21 gp75 vector (Kruskal-Wallis test, non-significant for both IgG1 and IgG2a, as compared to control groups that received LPS-activated B cells from wild-type mice that were either non-transduced (group B, figure 5) or transduced with MND-GFP (group D, Figure 5). As expected, the concentration of antibodies was significantly lower at day 24 when compared to mice that received p21-35 loaded, LPS-activated wild-type B cells (group A, Figure 5) (Mann-Whitney U test, p <0.002). The inability of the MND-p21gp75 transduced IL-10-/- B cells to inhibit the specific antibody response was not due to impaired homing of the genetically modified cells to (or their disappearance from) the lymphoid organs, as mRNA for p21gp75 from IL-10-/- B-cells could readily be detected by RT-PCR in the spleen of recipient mice 3 months post-transplantation, consistent with the long-term persistence of gene modified B cells in wild-type BALB/c mice (Figure 5D). This confirms that the elevated IL-10 production following transduction of B cells with MND-p21 gp75 was necessary to confer antigen-specific unresponsiveness in BALB/c mice.

The present data establish proof of concept that gene modified B cells presenting a peptide in the context of MHC class-II determinants induce antigen-specific and long-lasting state of unresponsiveness when such B cells are transferred either prior to or after immunization. To our knowledge, this is the first study showing that endosomal retargeting in B-cells can induce robust and unprecedented long-lasting immune tolerance in both prophylactic and therapeutic settings, even in the face of recurrent immunizations with adjuvant. Antigen-specific immune unresponsiveness correlated with constitutive production of IL-10 by transduced B cells. This required a novel mechanism that involves the cell surface recruitment of TLR2 along with the retroviral cellular receptor mCAT1 into lipid microrafts, ultimately leading to constitutive IL-10 production and subsequent induction of type 1-like regulatory T cells. Constitutive IL-10 induction was mediated by an epigenetic mechanism that required activation of the STAT-3 transcription factor and increased binding of phosphorylated histone 3 on the corresponding STAT-3 binding site in the IL-10 promoter. A summary scheme of this novel mechanism is provided in Figure 11.

### REFERENCES

- Brightbill, H. D., Plevy, S. E., Modlin, R. L., and Smale, S. T. (2000). A prominent role for Sp1 during lipopolysaccharide-mediated induction of the IL-10 promoter in macrophages. J Immunol 164, 1940-1951.
- Burke, F., Stagg, A. J., Bedford, P. A., English, N., and Knight, S. C. (2004). IL-10-producing B220+CD11 c- APC in mouse spleen. J Immunol 173, 2362-2372.
- Cassell, D. J., and Schwartz, R. H. (1994). A quantitative analysis of antigen-presenting cell function: activated B cells stimulate naive CD4 T cells but are inferior to dendritic cells in providing costimulation. J Exp Med 180, 1829-1840.
- Chandrashekran, A., Gordon, Y., and Colin, C. (2004). Targeted retroviral transduction of c-kit + hematopoietic cells using novel ligand display technology. Blood 104, 2697-2703.
- Chaussabel, D., and Banchereau, J. (2005). Dendritic cells, therapeutic vectors of immunity and tolerance. Am J Transplant 5, 205-206.
- El-Amine, M., Melo, M., Kang, Y., Nguyen, H., Qian, J., and Scott, D. W. (2000). Mechanisms of tolerance induction by a gene-transferred peptide-IgG fusion protein expressed in B lineage cells. J Immunol 165, 5631-5636.
- Fuchs, E. J., and Matzinger, P. (1992). B cells turn off virgin but not memory T cells. Science 258, 1156-1159.
- Gieni, R. S., Umetsu, D. T., and DeKruyff, R. H. (1997). Ly1- (CD5-) B cells produce interleukin (IL)-10. Cell Immunol 175, 164-170.
- Halene, S., Wang, L., Cooper, R. M., Bockstoce, D. C., Robbins, P. B., and Kohn, D. B. (1999). Improved expression in hematopoietic and lymphoid cells in mice after transplantation of bone marrow transduced with a modified retroviral vector. Blood 94, 3349-3357.
- Janssens, W., Carlier, V., Wu, B., VanderElst, L., Jacquemin, M. G., and Saint-Remy, J. M. (2003a). CD4(+)CD25(+) T cells lyse antigen-presenting B cells by Fas-Fas ligand interaction in an epitope-specific manner. J Immunol 171, 4604-4612.
- Janssens, W., Chuah, M. K., Naldini, L., Follenzi, A., Collen, D., Saint-Remy, J. M., and VandenDriessche, T. (2003b). Efficiency of onco-retroviral and lentiviral gene transfer into primary mouse and human B-lymphocytes is pseudotype dependent. Hum Gene Ther 14, 263-276.
- Korst, R. J., Mahtabifard, A., Yamada, R., and Crystal, R. G. (2002). Effect of adenovirus gene transfer vectors on the immunologic functions of mouse dendritic cells. Mol Ther 5, 307-315.
- Lee, W. C., Zhong, C., Qian, S., Wan, Y., Gauldie, J., Mi, Z., Robbins, P. D., Thomson, A. W., and Lu, L. (1998). Phenotype, function, and in vivo migration and survival of allogeneic dendritic cell progenitors genetically engineered to express TGF-beta. Transplantation 66, 1810-1817.
- Lei, T. C., and Scott, D. W. (2005). Induction of tolerance to factor VIII inhibitors by gene therapy with immunodominant A2 and C2 domains presented by B cells as Ig fusion proteins. Blood 105, 4865-4870.
- Lei, TC., Su Y. and Scott, D.W. (2005). Tolerance induction via a B-cell delivered gene therapy-based protocol: optimization and role of the Ig scaffold. Cell. Immunol. 235, 12-20.
- Liu, T. F., Kandala, G., and Setaluri, V. (2001). PDZ domain protein GIPC interacts with the cytoplasmic tail of melanosomal membrane protein gp75 (tyrosinase-related protein-1). J Biol Chem 276, 35768-35777.
- Lucas, M., Zhang, X., Prasanna, V., and Mosser, D. M. (2005). ERK activation following macrophage FcgammaR ligation leads to chromatin modifications at the IL-10 locus. J Immunol 175, 469-477.
- Mahnke, K., and Enk, A. H. (2005). Dendritic cells: key cells for the induction of regulatory T cells? Curr Top Microbiol Immunol 293, 133-150.
- Markowitz, D., Goff, S., and Bank, A. (1988). A safe packaging line for gene transfer: separating viral genes on two different plasmids. J Virol 62, 1120-1124.
- Masuda, M., Kakushima, N., Wilt, S. G., Ruscetti, S. K., Hoffman, P. M., and Iwamoto, A. (1999). Analysis of receptor usage by ecotropic murine retroviruses, using green fluorescent protein-tagged cationic amino acid transporters. J Virol 73, 8623-8629.
- Netea, M. G., Sutmuller, R., Hermann, C., Van der Graaf, C. A., Van der Meer, J. W., van Krieken, J. H., Hartung, T., Adema, G., and Kullberg, B. J. (2004). Toll-like receptor 2 suppresses immunity against Candida albicans through induction of IL-10 and regulatory T cells. J Immunol 172, 3712-3718.
- Panda, B. R., Kingsman, S. M., and Kingsman, A. J. (2000). Mutational analysis of the putative receptor-binding domain of Moloney murine leukemia virus glycoprotein gp70. Virology 273, 90-100.
- Riley, J. K., Takeda, K., Akira, S., and Schreiber, R. D. (1999). Interleukin-10 receptor signaling through the JAK-STAT pathway. Requirement for two distinct receptor-derived signals for anti-inflammatory action. J Biol Chem 274, 16513-16521.
- Roncarolo, M. G., Bacchetta, R., Bordignon, C., Narula, S., and Levings, M. K. (2001). Type 1 T regulatory cells. Immunol Rev 182, 68-79.
- Venditti, G., Di lanni, M., Falzetti, F., Moretti, L., Di Florio, S., and Tabilio, A. (2003). NeoR-based transduced T lymphocytes detected by real-time quantitative polymerase chain reaction. J Hematother Stem Cell Res 12, 83-91.
- Wu, B., Elst, L. V., Carlier, V., Jacquemin, M. G., and Saint-Remy, J. M. (2002). The Dermatophagoides pteronyssinus Group 2 Allergen Contains a Universally Immunogenic T Cell Epitope. J Immunol 169, 2430-2435.
- Wu, B., Toussaint, G., Vander Elst, L., Granier, C., Jacquemin, M. G., and Saint-Remy, J. M. (2000). Major T cell epitope-containing peptides can elicit strong antibody responses. Eur J Immunol 30, 291-299.
- Yang, L., Bailey, L., Baltimore, D., and Wang, P. (2006). Targeting lentiviral vectors to specific cell types in vivo. Proc. Natl. Acad. Sci. USA 103, 11479-11484.
- Zhang, X., Edwards, J. P., and Mosser, D. M. (2006). Dynamic and transient remodeling of the macrophage IL-10 promoter during transcription. J Immunol 177, 1282-1288.

## Claims

1. An in vitro method to condition B cells to produce IL-10, comprising activation of the TLR2 receptor, in combination with the activation of a second transmembrane protein.

2. The method of claim 1, whereby said second transmembrane protein is a transporter.

3. The method of claim 2, whereby said transporter acts as a retroviral receptor.

4. The method of claim 3, whereby said receptor is mCAT1

5. The method to any of the preceding claims, whereby the B cells is loaded with an antigen before, during or after the receptor activation.

## Patentansprüche

1. In-vitro-Verfahren zum Konditionieren von B-Zellen zur Herstellung von IL-10, wobei das Verfahren die Aktivierung des TLR2-Rezeptors in Kombination mit der Aktivierung eines zweiten Transmembranproteins umfasst.

2. Verfahren nach Anspruch 1, wobei es sich bei dem zweiten Transmembranprotein um einen Transporter handelt.

3. Verfahren nach Anspruch 2, wobei der Transporter als ein Retrovirusrezeptor wirkt.

4. Verfahren nach Anspruch 3, wobei es sich bei dem Rezeptor um mCAT1 handelt.

5. Verfahren nach einem der vorhergehenden Ansprüche, wobei die B-Zellen vor, während oder nach der Rezeptoraktivierung mit einem Antigen beladen werden.

## Revendications

1. Procédé in vitro pour conditionner des lymphocytes B à produire de l'IL-10, comprenant l'activation du récepteur TLR2 en combinaison avec l'activation d'une seconde protéine transmembranaire.

2. Procédé selon la revendication 1, par lequel ladite seconde protéine transmembranaire est un transporteur.

3. Procédé selon la revendication 2, par lequel ledit transporteur agit en tant que récepteur rétroviral.

4. Procédé selon la revendication 3, par lequel ledit récepteur est mCAT1.

5. Procédé selon l'une quelconque des revendications précédentes, par lequel les lymphocytes B sont chargés avec un antigène avant, pendant et après l'activation du récepteur.


### SEQUENCE LISTING

<110> VIB VZW
   LIFE SCIENCES RESEARCH PARTNERS VZW
<120> B CELL TOLERANCE INDUCTION
<130> TVD/TOL/v279
<150> US 60/978,343
   <151> 2007-10-08
<160> 13
<170> PatentIn version 3.5
<210> 1
   <211> 21
   <212> DNA
   <213> Artificial sequence
<220>
   <223> Primer
<400> 1
   ccctttatcc agccctcact c 21
<210> 2
   <211> 20
   <212> DNA
   <213> Artificial Sequence
<220>
   <223> Primer
<400> 2
   cctggggact ttccacaccc 20
<210> 3
   <211> 22
   <212> DNA
   <213> Artificial Sequence
<220>
   <223> Primer
<400> 3
   tcatgctggg atctgagctt ct 22
<210> 4
   <211> 23
   <212> DNA
   <213> Artificial Sequence
<220>
   <223> Primer
<400> 4
   cggaagtcac cttagcactc agt 23
<210> 5
   <211> 15
   <212> PRT
   <213> Artificial Sequence
<220>
   <223> Smartladder peptide
<400> 5
   Cys His Gly Ser Glu Pro Cys Ile Ile His Arg Gly Lys Pro Phe
   1               5                   10                  15
<210> 6
   <211> 15
   <212> PRT
   <213> Artificial sequence
<220>
   <223> 15-mer-peptide
<400> 6
   Asn Ala Cys His Tyr Met Lys Cys Pro Leu Val Lys Gly Gln Gln
   1               5                   10                  15
<210> 7
   <211> 20
   <212> DNA
   <213> Artificial Sequence
<220>
   <223> Probe
<400> 7
   ccggccgctt gggtggagag 20
<210> 8
   <211> 19
   <212> DNA
   <213> Artificial Sequence
<220>
   <223> Primer
<400> 8
   gatggattgc acgcaggtt 19
<210> 9
   <211> 21
   <212> DNA
   <213> Artificial Sequence
<220>
   <223> Primer
<400> 9
   gtgcccagtc atagccgaat a 21
<210> 10
   <211> 91
   <212> DNA
   <213> Artificial sequence
<220>
   <223> Primer
<400> 10
   ccggaattcc caccatggat tgccatggtc agaaccatgt atcattcatc gtggtaaacc 60
   attctctggt ggttccggag gttcaggagg c 91
<210> 11
   <211> 90
   <212> DNA
   <213> Artificial Sequence
<220>
   <223> Primer
<400> 11
   cagacaagaa gcaaccccga aaatggcagc tacaagtaac aacgcagcca ctacagcaat 60
   ggtaatgatg cctcctgaac ctccggaacc 90
<210> 12
   <211> 87
   <212> DNA
   <213> Artificial sequence
<220>
   <223> Primer
<400> 12
   gcatagcgtt gatagtgatc agtgaggaga ggctggttgg cttcattctt ggtgcttcta 60
   gaacggatca gacaagaagc aaccccg 87
<210> 13
   <211> 80
   <212> DNA
   <213> Artificial Sequence
<220>
   <223> Primer
<400> 13
   gtatctctcg agtcagacca tggagtggtt aggattcggg agctcctcat agtcctcagc 60
   atagcgttga tagtgatcag 80